# EUROPEAN PATENT APPLICATION

(11) **EP 4 733 414 A1**
(43) Date of publication of application: **29.04.2026**
(21) Application number: 24825754.5
(22) Date of filing: 07.06.2024
(51) Int. Cl.: C12Q 1/6883, C12Q 1/686, C12Q 1/6869, C12N 15/11

(54) **NOVEL METHOD FOR DETECTING ABNORMAL CELL HAVING GENE MUTATION**

(30) Priority: 20.06.2023 JP 2023100764
(71) Applicant: The University of Osaka, Osaka 565-0871 (JP)
(72) Inventor: NAKATA, Jun, Suita-shi, Osaka 565-0871 (JP); SUGIYAMA, Haruo, Suita-shi, Osaka 565-0871 (JP); MOTOOKA, Daisuke, Suita-shi, Osaka 565-0871 (JP); YAMANO, Hiroyuki, Suita-shi, Osaka 565-0871 (JP)
(74) Representative: Acapo Onsagers AS
(86) International application number: PCT/JP2024/020895
(87) International publication number: WO 2024/262348

(57) **Abstract**

A method for detecting abnormal cell having a genetic mutation, the method including: a step of amplifying, in genomic DNA derived from peripheral blood mononuclear cells of the patient, a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1 to obtain an amplification product; and a step of determining a nucleotide sequence of the amplification product and detecting a nucleotide mutation, in which the detection of the nucleotide mutation indicates that the lesion is present, and the amplification is performed by a PCR reaction using a primer having no additional sequence.

## Description

### TECHNICAL FIELD

The present invention relates to a novel method for detecting abnormal cell having a genetic mutation. Priority is claimed on Japanese Patent Application No. 2023-100764, filed June 20, 2023, the content of which is incorporated herein by reference.

### BACKGROUND ART

Acute myeloid leukemia is a disease group divided into about 20 subtypes. Acute myeloid leukemia is risk-classified based on its genetic background, and hematopoietic stem cell transplantation in the first complete remission is recommended for patients in intermediate- to high-risk group. On the other hand, in the low-risk group, only chemotherapy is recommended. However, relapse is observed in some cases even in the low-risk group.

In addition, even in the low-risk group, in a case where minimal residual disease (MRD) is detected during the treatment process, it is regarded as an independent risk factor for relapse and becomes a reason for selecting the hematopoietic stem cell transplantation in the first complete remission.

Various methods for detecting minimal residual disease are present. As a high-sensitivity assay system, a method of detecting WT1 mRNA (for example, see Non Patent Document 1), FACS-MRD (for example, see Non Patent Document 2), and the like are known.

### Citation List

### Non Patent Documents

Non-Patent Document 1: Inoue K., et al., Long-term follow-up of minimal residual disease in leukemia patients by monitoring WT1 (Wilms tumor gene) expression levels, Blood, 88 (6), 2267-2278, 1996.
Non-Patent Document 2: Campana D. and Coustan-Smith E., Detection of minimal residual disease in acute leukemia by flow cytometry, Cytometry, 38 (4), 139-152, 1999.
Non-Patent Document 3: Yasuda T., et al., Clinical utility of target capture-based panel sequencing in hematological malignancies: A multicenter feasibility study, Cancer Science, 111, 3367-3378, 2020.

### SUMMARY OF INVENTION

### Technical Problem

As one of the favorable prognostic acute myeloid leukemias, acute myeloid leukemia (WHO 4th edition disease name) with a mutation in both alleles of CEBPA or acute myeloid leukemia (WHO 5th edition disease name) with a mutation in the bZip region of CEBPA (hereinafter, referred to as "CEBPA-acute myeloid leukemia (AML)" or "CEBPA-AML") is known.

In CEBPA-AML, the WT1 mRNA expression level at the time of initial onset is low, and the WT1 mRNA expression becomes negative in almost all cases after one course of consolidation therapy has been administered. In addition, in the detection of minimal residual disease by FACS-MRD, although the relapse rate differs between positive cases and negative cases, the false-positive and false-negative rates have been reported to be about 30% to 50%, and the sensitivity and specificity are low.

In a retrospective cohort of a CEBPA-AML study performed by the present inventors, it was also shown that neither the WT1 mRNA expression level nor the detection of minimal residual disease by FACS-MRD had sufficient sensitivity and specificity for predicting relapse.

Therefore, an object of the present invention is to provide a detection technology for a lesion in a patient with acute myeloid leukemia.

### Solution to Problem

The present invention includes the following aspects.
[1] A method for detecting a lesion in a patient with acute myeloid leukemia, the method including a step of amplifying, in genomic DNA derived from peripheral blood mononuclear cells of the patient, a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1 to obtain an amplification product, and a step of determining a nucleotide sequence of the amplification product and detecting a nucleotide mutation, in which the detection of the nucleotide mutation indicates that the lesion is present, and the amplification is performed by a PCR reaction using a primer having no additional sequence.
[2] The detection method according to [1], in which the determination of the nucleotide sequence of the amplification product is performed by next-generation sequencing.
[3] The detection method according to [2], in which the next-generation sequencing is performed by paired-end sequencing.
[4] A kit for detecting a lesion in a patient with acute myeloid leukemia, the kit including a primer for amplifying a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1, in which the primer has no additional sequence.

### Advantageous Effects of Invention

According to the present invention, it is possible to provide a detection technology for minimal residual disease in a patient with acute myeloid leukemia after chemotherapy.

### BRIEF DESCRIPTION OF DRAWINGS

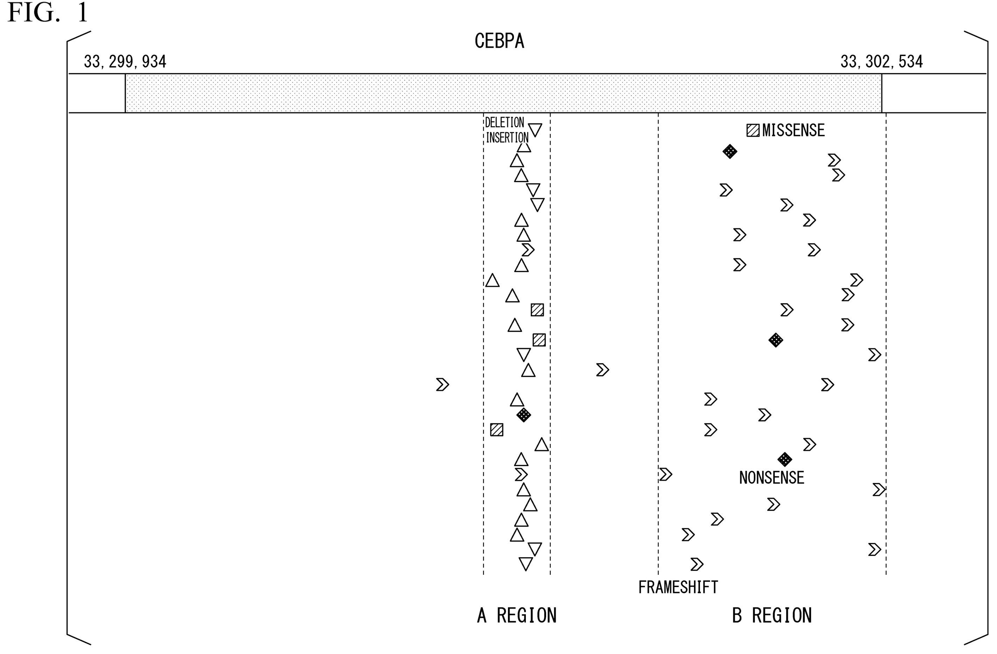

[FIG. 1] A schematic diagram of a CEBPA gene (genomic DNA).

### DESCRIPTION OF EMBODIMENTS

### [Method for detecting lesion in patient with acute myeloid leukemia]

In one embodiment, the present invention provides a method for detecting a lesion in a patient with acute myeloid leukemia, the method including a step of amplifying, in genomic DNA derived from peripheral blood mononuclear cells of the patient, a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1 to obtain an amplification product, and a step of determining a nucleotide sequence of the amplification product and detecting a nucleotide mutation, in which the detection of the nucleotide mutation indicates that the lesion is present, and the amplification is performed by a PCR reaction using a primer having no additional sequence. As will be described in Examples, the present inventors have clarified that the lesion in the patient with acute myeloid leukemia can be detected by the method according to the present embodiment.

Examples of the acute myeloid leukemia include acute myeloid leukemia (WHO 4th edition disease name) with a mutation both alleles of CCAAT Enhancer Binding Protein Alpha (CEBPA) and acute myeloid leukemia (WHO 5th edition disease name) with a mutation in the bZip region of CEBPA (CEBPA-AML). The CEBPA gene (genomic DNA) corresponds to a nucleotide sequence at positions 33,299,934 to 33,302,534 of the 19th chromosome (NCBI accession number: NC_000019.10) of the human reference genome Hg38.

In the method according to the present embodiment, the lesion in a patient with acute myeloid leukemia means cancerous cells in which a mutation is present in the CEBPA gene. That is, the detection of the lesion in the patient with acute myeloid leukemia means the detection of the presence of the cells having a mutation in the CEBPA gene.

In the method according to the present embodiment, a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1 in genomic DNA derived from peripheral blood mononuclear cells of the patient with acute myeloid leukemia is amplified to obtain an amplification product. In the present specification, a region corresponding to a certain nucleotide sequence refers to a region having a high homology in a case where the nucleotide sequence is subjected to a homology analysis (alignment analysis) with respect to the genomic DNA or a region mapped in a case where the nucleotide sequence is mapped to the genomic DNA. Here, the high homology means, for example, that the sequence identity between the compared sequences is 70% or more, preferably 80% or more, more preferably 90% or more, and still more preferably 95% or more. The region corresponding to a certain nucleotide sequence can also be referred to as a region defined by a certain nucleotide sequence. The SEQ ID No. 1 is a nucleotide sequence of the genomic DNA of the CEBPA gene. The nucleotide sequence set forth in SEQ ID NO: 1 corresponds to a nucleotide sequence at positions 33,299,934 to 33,303,000 of the NCBI accession number: NC_000019.10.

The peripheral blood mononuclear cells can be recovered from the blood derived from the patient by a method usually performed. In general, the peripheral blood mononuclear cells are recovered by a density gradient centrifugation method using a commercially available lymphocyte separation solution.

FIG. 1 is a schematic diagram of a CEBPA gene (genomic DNA). In FIG. 1, the numbers in the upper left and upper right indicate positions in the nucleotide sequence of the NCBI accession number: NC_000019.10. In addition, each row shown in FIG. 1 corresponds to a case of the CEBPA-AML patient. A region corresponding to a region including a nucleotide sequence at positions 1,316 to 1,663 of SEQ ID NO: 1 is defined by an A region, and a region corresponding to a region including a nucleotide sequence at positions 1,931 to 2,642 of SEQ ID NO: 1 is defined by a B region.

As shown in FIG. 1, in the CEBPA-AML patient, two types of mutations, that is, a mutation in the A region and a mutation in the B region, are present for each case, and the contents of the mutations are different from each other. Therefore, it is difficult to design a primer for quantitative real-time PCR for detecting the lesion in the patient with acute myeloid leukemia.

In addition, the A region and the B region of the CEBPA gene on the genomic DNA are GC-rich. Therefore, in a case where these regions are PCR-amplified, abnormal PCR products are generated. In a case of PCR amplification, it is often performed to add an additional nucleotide sequence for a molecular barcode or an additional nucleotide sequence for secondary PCR to the primer. On the other hand, the present inventors have found that the occurrence of abnormal PCR products can be suppressed by performing the PCR amplification of the region (A region) corresponding to a region including a nucleotide sequence at positions 1,316 to 1,663 of SEQ ID NO: 1 and/or the region (B region) corresponding to a region including a nucleotide sequence at positions 1,931 to 2,642 of SEQ ID NO: 1 by a PCR reaction using a primer having no additional sequence. The primer having no additional sequence means a primer that does not have no additional sequence. That is, it means that the entire primer is complementary to the genomic DNA. After the amplification of the genomic DNA, the PCR amplification using the primer having an additional sequence may be performed. Examples of the additional sequence include an adapter sequence for next-generation sequencing described below.

The present inventors have also found that the mutations observed in the case of the CEBPA-AML patient are often deletion mutations or insertion mutations, and these mutations are hardly observed in abnormal PCR products. Therefore, by performing the PCR amplification of the genomic DNA by a PCR reaction using a primer without an additional sequence and determining the nucleotide sequence of the amplification product, the nucleotide sequence of the CEBPA gene in the genomic DNA derived from the peripheral blood mononuclear cells of the CEBPA-AML patient can be determined, and the nucleotide mutation in the A region and the B region or the nucleotide mutation in the A region or the B region can be detected.

The method according to the present embodiment may be used for the diagnosis of acute myeloid leukemia (CEBPA-AML). As will be described in Examples, by the method according to the present embodiment, acute myeloid leukemia can be diagnosed with higher accuracy than the conventional capture panel method.

Alternatively, the method according to the present embodiment may be used for detecting a lesion in a patient with acute myeloid leukemia after chemotherapy. Here, the lesion may be minimal residual disease (MRD). As will be described in Examples, the detection result of minimal residual disease after chemotherapy by the method according to the present embodiment is correlated with the relapse rate of acute myeloid leukemia. That is, since the relapse of acute myeloid leukemia can be predicted by the method according to the present embodiment, it is possible to provide a basis for determining whether or not to select hematopoietic stem cell transplantation in the first complete remission.

In the method of the present embodiment, it is preferable that the determination of the nucleotide sequence of the amplification product is performed by next-generation sequencing. By using next-generation sequencing, it is possible to significantly reduce the effort required for sequencing.

Next-generation sequencing is preferably performed by paired-end sequencing. In paired-end sequencing, since the amplification product is sequenced from both ends and joined, more accurate sequencing can be performed. In addition, to reduce a sequencing error, it is preferable that a nucleotide sequence that cannot be sequenced well is removed by a program.

The length (read length) of the nucleotide sequence that can be determined by next-generation sequencing is about 200 bp. Therefore, it is preferable that the primer is selected such that the length of the amplification product of the A region and/or the B region is 200 bp or less. In a case of performing paired-end sequencing, it is preferable that the primer is selected such that the length of the amplification product of the A region and/or the B region is 400 bp or less.

The region (A region) corresponding to a region including a nucleotide sequence at positions 1,316 to 1,663 of SEQ ID NO: 1 may be amplified by PCR amplification using a single set of primers. On the other hand, since the region (B region) including a nucleotide sequence at positions 1,931 to 2,642 of SEQ ID NO: 1 is 600 bp or more, it is preferable to divide the region into two or more regions and amplify the divided regions. In addition, the PCR amplification of the A region and/or the B region may be performed using a plurality of primer sets, and the nucleotide mutation may be checked a plurality of times.

The primer set for amplifying the A region is not particularly limited as long as it can amplify the region, and specific examples thereof include a set of primers of 5'-GGCCAAGAAGTCGGTGGACA-3' (SEQ ID NO: 2) and 5'-CTCGGGCAAGCCTCGAGAT-3' (SEQ ID NO: 3). The set of the primer of SEQ ID NO: 2 and the primer of SEQ ID NO: 3 can amplify a region of the nucleotide sequence at positions 33,301,249 to 33,301,596 of the CEBPA gene (nucleotide sequence of NCBI accession number: NC_000019.10) of the genomic DNA.

The primer set for amplifying the B region is not particularly limited as long as it can amplify the region, and specific examples thereof include a combination of a set of primers of 5'-TCCATCGACATCAGCGCCTA-3' (SEQ ID NO: 4) and 5'-GGCGGCTGGTAAGGGAAGAG-3' (SEQ ID NO: 5) and a set of primers of 5'-GGCCTGCCGGGTATAAAAGC-3' (SEQ ID NO: 6) and 5'-GCCAGGAACTCGTCGTTGAA-3' (SEQ ID NO: 7).

The set of the primer of SEQ ID NO: 4 and the primer of SEQ ID NO: 5 can amplify a region of the nucleotide sequence at positions 33,301,864 to 33,302,234 of the CEBPA gene (nucleotide sequence of NCBI accession number: NC_000019.10) of the genomic DNA.

The set of the primer of SEQ ID NO: 6 and the primer of SEQ ID NO: 7 can amplify a region of the nucleotide sequence at positions 33,302,179 to 33,302,575 of the CEBPA gene (nucleotide sequence of NCBI accession number: NC_000019.10) of the genomic DNA. That is, the set of the primers of SEQ ID NOs. 4, 5, 6, and 7 divides the B region into two regions and amplifies the divided regions.

### [Kit for detecting lesion in patient with acute myeloid leukemia]

In one embodiment, the present invention provides a kit for detecting a lesion in a patient with acute myeloid leukemia, the kit including a primer for amplifying a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1, in which the primer has no additional sequence. By the kit according to the present embodiment, the above-described method for detecting a lesion in a patient with acute myeloid leukemia can be suitably performed.

In the kit according to the present embodiment, the patient with acute myeloid leukemia, the lesion, the primer not including an additional sequence, and the like are as described above.

The primer set for amplifying the region (A region) corresponding to a region including a nucleotide sequence at positions 1,316 to 1,663 of SEQ ID NO: 1 is not particularly limited as long as it can amplify this region, and specific examples thereof include the set of the primer of SEQ ID NO: 2 and the primer of SEQ ID NO: 3 described above.

The specific primer set for amplifying the region corresponding to the region (B region) including the nucleotide sequence at positions 1,931 to 2,642 of SEQ ID NO: 1 is not particularly limited as long as it can amplify the region, and specific examples thereof include a combination of the set of the primer of SEQ ID NO: 4 and the primer of SEQ ID NO: 5 described above and the set of the primer of SEQ ID NO: 6 and the primer of SEQ ID NO: 7 described above.

The kit according to the present embodiment may include one of a primer set for amplifying a region corresponding to the A region or a primer set for amplifying a region corresponding to the B region, or may include one of the primer set for amplifying a region corresponding to the A region and the primer set for amplifying a region corresponding to the B region.

### [Other Embodiments]

In one embodiment, the present invention provides a method for treating a patient with acute myeloid leukemia, the treatment method comprising: (a) a step of amplifying, in genomic DNA derived from peripheral blood mononuclear cells of the patient, a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1 to obtain an amplification product, in which the amplification is performed by a PCR reaction using a primer having no additional sequence; (b) a step of determining a nucleotide sequence of the amplification product and detecting a nucleotide mutation, in which the detection of the nucleotide mutation indicates that the lesion is present in a patient with acute myeloid leukemia; and (c) a step of performing hematopoietic stem cell transplantation on the patient in the first complete remission in a case where the nucleotide mutation is detected.

In the treatment method according to the present embodiment, the patient with acute myeloid leukemia, the lesion, the primer not including an additional sequence, and the like are as described above.

### Examples

Next, the present invention will be described in more detail by way of Examples; however, the present invention is not intended to be limited to the following Examples.

### [Experimental Example 1]

### (Detection of lesion using sample derived from patient with acute myeloid leukemia)

Peripheral blood mononuclear cells were recovered from a blood sample of a CEBPA-AML patient (at the time of initial onset), and DNA was extracted. Subsequently, a PCR reaction was performed using the set of the primer of SEQ ID NO: 2 and the primer of SEQ ID NO: 3, the set of the primer of SEQ ID NO: 4 and the primer of SEQ ID NO: 5, and the set of the primer of SEQ ID NO: 6 and the primer of SEQ ID NO: 7, thereby obtaining amplification products. The primers of SEQ ID NOs: 2, 3, 4, 5, 6, and 7 had the same nucleotide sequence as the genomic DNA and were primers without an additional sequence.

As described above, by PCR using the set of the primer of SEQ ID NO: 2 and the primer of SEQ ID NO: 3, a region (region including a nucleotide sequence at positions 1,316 to 1,663 of SEQ ID NO: 1, region including A region) including the nucleotide sequence (nucleotide sequence of NCBI accession number: NC_000019.10) at positions 33,301,249 to 33,301,596 of the genomic DNA of the CEBPA gene can be amplified.

In addition, by PCR using the set of the primer of SEQ ID NO: 4 and the primer of SEQ ID NO: 5, a region (region including a nucleotide sequence at positions 1,931 to 2,301 of SEQ ID NO: 1, a part of B region) including the nucleotide sequence (nucleotide sequence of NCBI accession number: NC_000019.10) at positions 33,301,864 to 33,302,234 of the genomic DNA of the CEBPA gene can be amplified.

In addition, by PCR using the set of the primer of SEQ ID NO: 6 and the primer of SEQ ID NO: 7, a region (region including a nucleotide sequence at positions 2,246 to 2,642 of SEQ ID NO: 1, a part of B region) including the nucleotide sequence (nucleotide sequence of NCBI accession number: NC_000019.10) at positions 33,302,179 to 33,302,575 of the genomic DNA of the CEBPA gene can be amplified.

Subsequently, using a commercially available kit (product name "Kapa Hyper Prep Kit", Kapa Biosystems), a sequencing adapter sequence was added to the amplification product. Subsequently, paired-end sequencing was performed using a sequencer (product name "MiSeq", Illumina).

Tables 1 and 2 below show a case number, a mutation position, a mutation detail, and a mutation detection result of patient with CEBPA-AML. (A) after the case number indicates a mutation in the A region. In addition, (B) after the case number indicates a mutation in the B region.

In addition, the mutation position indicates a position on the 19th chromosome (NCBI accession number: NC_000019.10) of the human reference genome Hg38. In addition, the mutation detail indicates a specific mutation at the mutation position. Furthermore, in the detection result, a denominator represents the number of reads that have been successfully subjected to pair-end sequencing, and the numerator represents, among them, the number of reads having the CEBPA mutation in each case.

In the present experimental example, since the sample at the time of initial onset of CEBPA-AML was used, the read having the mutation was detected at a ratio of about 50%.

**[Table 1]**

| Case | Mutation position | Mutation detail | SEQ ID NO | Detection result |
|---|---|---|---|---|
| Case 55(A) | 33301480 | -GCTGCGTCTCCACGTTGC | 8 | 47318/95410 |
| Case 65(A) | 33301481 | +CTGCGT | - | 39580/81457 |
| Case 77(A) | 33301468 | +TGG | - | 38088/80510 |
| Case 107(A) | 33301475 | +CTT | - | 37543/81556 |
| Case 148(A) | 33301480 | -GCTGCGTCTCCACGTTGC | 9 | 41334/87424 |
| Case 194(A) | 33301475 | +CTT | - | 35085/75073 |
| Case 202(A) | 33301478 | +CTG | - | 34363/75076 |
| Case 244(A) | 33301487 | +CTCCACGTTGCGCTG | 10 | 30982/70317 |
| Case 251(A) | 33301475 | +CTT | - | 36743/86201 |
| Case 259(A) | 33301482 | +TGCGTCTCCACGTTGCGCTGCTTGGC CTTG | 11 | 21468/54984 |
| Case 318(A) | 33301462 | +TCA | - | 28455/65453 |
| Case 334(A) | 33301498 | G | - | 32873/81398 |
| Case 349(A) | 33301466 | +CTCCAGCACCTTCTGCTGCGTCTCCA CGTTGCT | 12 | 23890/65676 |
| Case 362(A) | 33301501 | G | - | 23863/55623 |
| Case 379(A) | 33301478 | -CTG | - | 21593/69670 |
| Case 382(A) | 33301484 | +CTT | - | 39994/89158 |
| Case 417(A) | 33301371 | +CT | - | 18687/71563 |
| Case 427(A) | 33301478 | +CTG | - | 29957/74692 |
| Case 456(A) | 33301469 | +CAG | - | 33173/74597 |
| Case 482(A) | 33301444 | G | - | 25495/78773 |
| Case 524(A) | 33301475 | +CTT | - | 35635/78391 |
| Case 548(A) | 33301475 | +CTT | - | 37440/78852 |
| Case 567(A) | 33301494 | +TTGCGCTGC | - | 42978/88391 |
| Case 575(A) | 33301477 | +TCTGCTGCGTCTCCACGTTGCGCTGC TTGG | 13 | 30380/79540 |
| Case 593(A) | 33301486 | +TCT | - | 44804/104237 |
| Case 619(A) | 33301475 | +CTT | - | 34645/71860 |
| Case 646(A) | 33301469 | +CAG | - | 32728/71744 |
| Case 660(A) | 33301493 | -GTT | - | 41533/85130 |
| Case 664(A) | 33301478 | -CTGCTG | - | 49258/101678 |
| Case 707(A) | 33301500 | GG | - | 40672/81399 |
| Case 713(A) | 33301498 | +GCT | - | 41310/86633 |

**[Table 2]**

| Case | Mutation position | Mutation detail | SEQ ID NO | Detection result |
|---|---|---|---|---|
| Case 55(B) | 33302180 | -CAGG | - | 22634/46118 |
| Case 65(B) | 33302156 | A | - | 17081/35762 |
| Case 77(B) | 33302293 | +GCGCGGG | - | 20527/45119 |
| Case 107(B) | 33302289 | -GGCTGCGCGG | 14 | 23136/49057 |
| Case 148(B) | 33302225 | -GTCGATGGAC | 15 | 23183/50551 |
| Case 194(B) | 33302252 | -GCCGCCCAGC | 16 | 20890/44243 |
| Case 202(B) | 33302167 | -G | - | 14832/31026 |
| Case 244(B) | 33302069 | +G | - | 13315/31774 |
| Case 251(B) | 33302271 | +GC | - | 22358/46382 |
| Case 259(B) | 33302167 | -G | - | 17127/37429 |
| Case 318(B) | 33302346 | -G | - | 16891/36074 |
| Case 334(B) | 33302323 | -AG | - | 20429/44708 |
| Case 349(B) | 33302304 | -GCGCCCCG | - | 16700/37185 |
| Case 362(B) | 33302231 | +GGAC | - | 19023/48169 |
| Case 379(B) | 33302311 | -G | - | 17207/38587 |
| Case 382(B) | 33302214 | +C | - | 14845/57197 |
| Case 417(B) | 33302346 | -G | - | 12816/40591 |
| Case 427(B) | 33302069 | -GGGGCCCGCG | 17 | 16940/36192 |
| Case 456(B) | 33302285 | -G | - | 19915/40944 |
| Case 482(B) | 33302129 | +CA | - | 7614/24757 |
| Case 524(B) | 33302228 | +A | - | 13569/39517 |
| Case 548(B) | 33302060 | -GGCGCCGCCG | 18 | 10697/22077 |
| Case 567(B) | 33302352 | +CT | - | 22030/47274 |
| Case 575(B) | 33302213 | +GTAG | - | 24363/75284 |
| Case 593(B) | 33302136 | -G | - | 8991/19528 |
| Case 619(B) | 33302096 | -A | - | 8845/20941 |
| Case 646(B) | 33302346 | -G | - | 21588/45105 |
| Case 660(B) | 33302112 | +C | - | 11471/21595 |
| Case 664(B) | 33302280 | +G | - | 21841/45668 |
| Case 707(B) | 33302205 | -G | - | 31586/66184 |
| Case 713(B) | | | | |

As a genetic mutation test performed at the time of initial diagnosis of acute myeloid leukemia, a capture panel method (for example, see Non Patent Document 3) and the like have been known in the related art. The capture panel method is a method of fragmenting the genomic DNA to about 150 to 250 bases, recovering a gene region of interest using a complementary probe, and sequencing the recovered gene region with a next-generation sequencer. In the capture panel method, it is, in principle, difficult to detect long insertions or deletions in nucleotide sequences. The present inventors have found that, in the genetic mutation test by the capture panel method, a mutation was recognized only in the B region of CEPBA, but in one case considered to be CEBPA-AML with a mutation in both the A region and the B region from the clinical course and the surface phenotype, it was possible to confirm that the A region had a 45-nucleotides insertion mutation by the method of the present experimental example. This result indicates that CEBPA-AML can be diagnosed by the method of the present experimental example with higher accuracy than the capture panel method.

The above results indicate that the lesion in the patient with acute myeloid leukemia can be detected by amplifying the genomic DNA of the patient with acute myeloid leukemia by a PCR reaction using a primer without an additional sequence and sequencing the amplification product with a next-generation sequencer.

### [Experimental Example 2]

### (Study on detection accuracy of lesion)

The sample derived from the patient with acute myeloid leukemia was diluted and the detection accuracy of the lesion was studied. Specifically, peripheral blood mononuclear cells were recovered from a blood sample of a CEBPA-AML patient (case 107, at the time of initial onset), and the peripheral blood mononuclear cells derived from the healthy person were mixed thereto such that the cells derived from the patient were 10%, 1%, 0.1%, and 0.01%. In addition, as a negative control, peripheral blood mononuclear cells derived from a healthy person, in which the cells derived from the patient did not mixed, were used.

Subsequently, DNA was extracted from each cell, and the B region of the CEBPA gene in the genomic DNA was amplified by PCR using the set of the primer of SEQ ID NO: 6 and the primer of SEQ ID NO: 7.

Subsequently, using a commercially available kit (product name "Kapa Hyper Prep Kit", Kapa Biosystems), a sequencing adapter sequence was added to the amplification product. Subsequently, paired-end sequencing was performed using a sequencer (product name "MiSeq", Illumina).

Table 3 shows the results of sequencing and analyzing about 20,000 reads to detect a mutation. In Table 3, in the detection result, a denominator represents the number of reads that have been successfully subjected to pair-end sequencing, and the numerator represents, among them, the number of reads having the CEBPA mutation. As a result, the detected mutation decreased in proportion to the mixing ratio of the cells derived from the patient.

**[Table 3]**

| Sample | Detection result | Detection ratio (%) |
|---|---|---|
| Case 107, 10% | 767/22669 | 3.383 |
| Case 107, 1% | 55/19929 | 0.275 |
| Case 107, 0.1% | 11/26913 | 0.040 |
| Case 107, 0.01% | 0/20798 | 0 |
| Negative control | 0/24133 | 0 |

Subsequently, for the sample in which the cells derived from the patient were 0.1% and 0.01%, about 400,000 reads were sequenced and analyzed to detect a mutation.

Table 4 shows the results. In Table 4, in the detection result, a denominator represents the number of reads that have been successfully subjected to pair-end sequencing, and the numerator represents, among them, the number of reads having the CEBPA mutation.

**[Table 4]**

| Sample | Detection result | Detection ratio (%) |
|---|---|---|
| Case 107, 0.1% | 98/412601 | 0.0237 |
| Case 107, 0.01% | 8/365410 | 0.0021 |
| Negative control | 0/364366 | 0 |

As a result, in the sample derived from the healthy person, which was a negative control, no mutation was detected, whereas in the sample in which the cells derived from the patient were 0.1% and 0.01%, the mutation was detected at a ratio of 0.02% and 0.002%.

This result indicates that the present detection system can detect a mutation with high specificity even in a case where the ratio of the mutated cells is about 0.01%.

### [Experimental Example 3]

### (Study on prognosis of patient with acute myeloid leukemia after chemotherapy)

The minimal residual disease in the patient with acute myeloid leukemia during and after the chemotherapy was detected by the same method as in Experimental Example 1. As a result, the disappearance time or the persistent residual of the minimal residual disease during the treatment process could be followed. This indicates that it is possible to determine whether or not further additional treatment is required by the detection during the treatment. In addition, most of the patients who were positive for minimal residual disease after the final chemotherapy relapsed, whereas the patients who were negative for minimal residual disease after the final chemotherapy hardly relapsed, and the significant difference in the relapse-free survival ratio between the two groups was found to be P < 0.01 by the Wilcoxon method.

### INDUSTRIAL APPLICABILITY

According to the present invention, it is possible to provide a detection technology for a lesion in a patient with acute myeloid leukemia. According to the present detection technology, it is possible to diagnose acute myeloid leukemia with a CEBPA mutation with higher accuracy than the conventional method (capture panel method). In addition, the prognosis of the patient with acute myeloid leukemia after the chemotherapy can be predicted, and it can be used as a basis for determining additional treatment such as hematopoietic stem cell transplantation.

## Claims

1. A method for detecting a lesion in a patient with acute myeloid leukemia, the method comprising:
a step of amplifying, in genomic DNA derived from peripheral blood mononuclear cells of the patient, a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1 to obtain an amplification product; and
a step of determining a nucleotide sequence of the amplification product and detecting a nucleotide mutation,
wherein the detection of the nucleotide mutation indicates that the lesion is present, and
the amplification is performed by a PCR reaction using a primer having no additional sequence.

2. The detection method according to claim 1,
wherein the determination of the nucleotide sequence of the amplification product is performed by next-generation sequencing.

3. The detection method according to claim 2,
wherein the next-generation sequencing is performed by paired-end sequencing.

4. A kit for detecting a lesion in a patient with acute myeloid leukemia, the kit comprising:
a primer for amplifying a region corresponding to a region including a nucleotide sequence at positions 1,316 to 1,663 of SEQ ID NO: 1 and/or a region corresponding to a region including a nucleotide sequence at positions 1,931 to 2,642 of SEQ ID NO: 1,
wherein the primer has no additional sequence.
